# EUROPEAN PATENT APPLICATION

(11) **EP 2 045 239 A1**
(43) Date of publication of application: **08.04.2009**
(21) Application number: 07788619.0
(22) Date of filing: 20.06.2007
(51) Int. Cl.: C07D 209/48

(54) **NOVEL DERIVATIVES OF PHTHALIMIDE AS HISTONE DEACETYLASE INHIBITORS**

(30) Priority: 07.07.2006 ES 200601945
(71) Applicant: Universidad De Granada, 18071 Granada (ES)
(72) Inventor: GOMEZ VIDAL, Jose, Antonio, E-18071 Granada (ES); DOMINGUEZ SEGLAR, Jose, Francisco, E-18071 Granada (ES); TABRAUE CHAVEZ, Mavys, E-18071 Granada (ES)
(74) Representative: Carpintero Lopez, Francisco
(86) International application number: PCT/ES2007/000371
(87) International publication number: WO 2008/003801

(57) **Abstract**

The invention relates to novel compounds of general formula (I), or one of the salts thereof, particularly one of the pharmaceutically acceptable salts thereof, or one of the corresponding solvates thereof. These compounds are inhibitors of the histone deacetylase enzymes and are suitable as pharmacologically active agents in a medicament for the treatment and/or prophylaxis of disorders or diseases associated with histone deacetylases. The invention also relates to a process for obtaining the mentioned compounds and the pharmaceutical compositions containing them.

## Description

### Field of the Art

The present invention relates to new histone deacetylase inhibitor compounds, to new pharmaceutical compositions comprising them, and to processes for obtaining them. These compounds are suitable as pharmacologically active agents in a medicament for the treatment and/or prophylaxis of diseases related to histone deacetylases.

### Background of the Invention

The human genome is located inside the cell nucleus in chromatin, which is a dynamic macromolecular complex formed by nucleosomes. A single nucleosome is made up of a DNA fragment (146 base pairs) coiled around a histone octamer. Histones are small basic proteins rich in the amino acids lysine and arginine. The four types of nucleosomal histones contain two domains: the C-terminal domain, located inside the nucleosome and the N-terminal domain with lysine residues extending outside it.

The acetylation of lysine residues in these N-terminal sequences is mediated by the enzymes called histone acetyltransferases (HATs). The acetyl groups are eliminated from ε-N-acetyl-lysines by the activity of histone deacetylases (HDACs). The activities of HATs and HDACs are associated to the target genes through complexes formed by specific transcription factors for certain sequences and their respective cofactors. The balance between the opposite activities of HATs and HDACs regulates the acetylation state of histones [Marks, P. A.; Richon, V. M.; Rifkind, R. A. Histone deacetylase inhibitors: inducers of differentiation or apoptosis of transformed cells. J. Natl. Cancer Inst. 2000, 92, 1210-1216]. This type of modification regulates key essential processes in the cell in response to extracellular signals [a) Marks, P. A.; Rifkind, R. A.; Richon, V. M.; Breslow, R.; Miller, T.; Kelly, W. K. Histone deacetylases and cancer: causes and therapies. Nature Reviews Cancer 2001, 1 (3), 194-202; b) Workman, P. Scoring a bull's-eye against cancer genome targets. Curr. Op. Pharmacol. 2001, 1, 342-352].

High acetylation levels (hyperacetylation) are generally associated to an increase in transcriptional activity, whereas low acetylation levels (hypoacetylation) are associated to the repression of gene expression.

The family of HDACs in mammals includes three sub-classes [Gray, S. G. Ekström, T. J. The human histone deacetylase family. Exp. Cell Res. 2001, 262, 75-83]. Class I includes the HDAC1, HDAC2, HDAC3 and HDAC8 isoforms. Class includes the HDAC4, HDAC, HDAC6, HDAC7, HDAC9 and HDAC10 isoenzymes. Finally, class III is homologous to the sir2 yeast protein and includes NAD+-dependent SIRT1-7 isoenzymes and are known as sirtuins. HDAC11 has also been identified as a new member of the family of HDACs, but given the little sequential similarity with the rest, it is not classified within the previous classes. The large number of HDAC isoenzymes and of proteins that interact allows modulating the specificity of the substrate and even modifying the selectivity towards non-histone type targets.

Histone deacetylase enzyme inhibitors which can re-activate gene expression and inhibit tumor cell growth are known in the state of the art, therefore their use in the treatment against cancer is investigated.

Thus, some first-generation histone deacetylase inhibitors are being studied in phase I and II clinical trials [a) Minucci, S.; Pelicci, P. G. Histone deacetylase inhibitors and the promise of epigenetic (and more) treatments for cancer. Nature Reviews Cancer 2006, 6(1), 38-51; b) Johnstone, R. W. Histone-deacetylase inhibitors: novel drugs for the treatment of cancer. Nature Reviews Drug Discovery 2002, 1 (4), 287-299; c) Mai, A.; Massa, S.; Rotili, D.; Cerbara, II.; Valente, S.; Pezzi, R.; Simeoni, S.; Ragno, R. Histone deacetylation in epigenetics: An attractive target for anticancer therapy. Medicinal Research Reviews 2005, 25(3), 261-309]. Due to the fact that the most recently discovered HDAC inhibitors seem to overcome many of the most negative aspects of first-generation inhibitors in clinical use, the therapeutic value derived from the inhibition of HDACs in leukemias and other diseases, including solid and altered hormonal signal-dependent tumors, can be established [Krämer, O. H.; Göttlicher, M.; Heinzel, T. Histone deacetylase as a therapeutic target. Trends Endocrinol. Metabol. 2001, 12, 294-300].

Although this type of inhibitor was initially developed for the treatment of cancer, its use has been proposed in another king of proliferative-type diseases, such as psoriasis [McLaughlin, F.; La Thangue, N. B. Histone deacetylase inhibitors in psoriasis therapy. Current Drug Targets: Inflammation & Allergy 2004, 3(2), 213-219].

The use of this type of inhibitor in the treatment of inflammatory type diseases has also been described [Blanchard, F.; Chipoy, C. Histone deacetylase inhibitors: new drugs for the treatment of inflammatory diseases? Drug Discovery Today 2005, 10(3), 197-204].

A combined therapy with histone deacetylase inhibitors in the treatment against HIV has recently been proposed [Imai, K.; Okamoto, T. Transcriptional Repression of Human Immunodeficiency Virus Type 1 by AP-4. Journal of Biological Chemistry 2006, 281 (18), 12495-12505].

Histone deacetylase inhibitors are also useful for the treatment of Alzheimer's disease and dementia [Beglopoulos, V.; Shen, J. Regulation of CRE-dependent transcription by presenilins: prospects for therapy of Alzheimer's disease. Trends in Pharmacological Sciences 2006, 27(1), 33-40].

It would therefore be desirable to identify new histone deacetylase enzyme inhibitor compounds for their use in the treatment or prophylaxis of diseases in which the inhibition of said HDAC enzymes is involved.

The present invention faces the problem of providing alternative histone deacetylase inhibitors to those existing in the state of the art. It has surprisingly been discovered that hydroxamic acid derivative compounds of general formula (I) set forth below have a good affinity for histone deacetylases, causing their inhibition. Therefore, these compounds are particularly suitable as pharmacologically active agents in a medicament for the treatment and/or prophylaxis of disorders or diseases sensitive to the inhibition of histone deacetylase enzymes.

### Object of the Invention

In one aspect, the present invention provides a compound of general formula 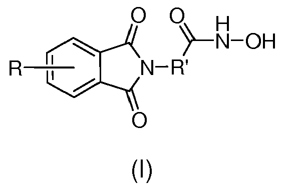 wherein
R' represents a -(CH₂)ₙ- radical wherein n is 5 or 6 or a 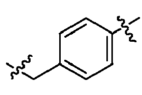 radical and R represents hydrogen, C1-C3 alkyl, phenyl, benzyl, F, Cl, Br, -OR1 wherein R1 represents hydrogen, C1-C3 alkyl, phenyl or benzyl, -NO₂, an amine of general formula -NR₁R₂ wherein R₁ and R₂, the same or different, can be hydrogen, C1-C3 alkyl, phenyl, or benzyl, or an -NHCOR₃ radical wherein R₃ can be C1-C3 alkyl, phenyl or benzyl,
optionally in the form of one of the salts thereof, particularly one of the pharmaceutically acceptable salts thereof, or one of the corresponding solvates thereof, with the proviso that when R is hydrogen, R' is different from -(CH₂)ₙ-wherein n is 5 or 6.

The compounds of general formula (I) have affinity for the histone deacetylase enzymes and are inhibitors thereof. They are useful in the production of medicaments which are suitable for the treatment and/or prophylaxis of disorders or diseases sensitive to the inhibition of histone deacetylases.

Therefore in another additional aspect, the present invention provides a pharmaceutical composition comprising at least one compound of general formula (I) or one of the pharmaceutically acceptable salts thereof, or one of the corresponding solvates thereof.

Likewise, the present invention provides a compound of general formula (I) or one of the pharmaceutically acceptable salts thereof, or one of the corresponding solvates thereof for the treatment and/or prophylaxis of diseases sensitive to the inhibition of histone deacetylases in a mammal, including a human being.

In an additional aspect, the present invention provides a compound of general formula (I) or one of the pharmaceutically acceptable salts thereof, or one of the corresponding solvates thereof for the treatment and/or prophylaxis of cancer, inflammatory type diseases, psoriasis, Alzheimer's disease, senile dementia or infection caused by the human immunodeficiency virus (HIV) in a mammal, including a human being.

In another aspect, the present invention provides the use of a compound of general formula (I) or one of the pharmaceutically acceptable salts thereof, or one of the corresponding solvates thereof, in the production of a medicament for the treatment and/or prophylaxis of diseases sensitive to the inhibition of histone deacetylases. In an additional aspect, the present invention provides the use of a compound of general formula (I) or one of the pharmaceutically acceptable salts thereof, or one of the corresponding solvates thereof, in the production of a medicament for the treatment and/or prophylaxis of cancer, inflammatory type diseases, psoriasis, Alzheimer's disease, senile dementia or infection caused by the human immunodeficiency virus (HIV) in a mammal, including a human being.

In a final aspect, the present invention provides processes for preparing a compound of general formula (I) as has been described above.

### Description of the Invention

In one aspect, the present invention provides a compound of general formula 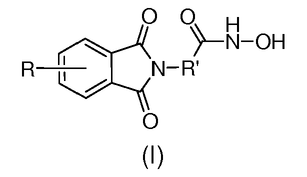 wherein
R' represents a -(CH₂)ₙ- radical wherein n is 5 or 6 or a 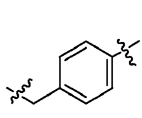 radical and R represents hydrogen, C1-C3 alkyl, phenyl (Ph), benzyl (Bn), F, Cl, Br, -OR1 wherein R1 represents hydrogen, C1-C3 alkyl, phenyl or benzyl, -NO₂, an amine of general formula -NR₁R₂ wherein R₁ and R₂, the same or different, can be hydrogen, C1-C3 alkyl, phenyl, or benzyl, or an -NHCOR₃ radical wherein R₃ can be C1-C3 alkyl, phenyl or benzyl,
optionally in the form of one of the salts thereof, particularly one of the pharmaceutically acceptable salts thereof, or one of the corresponding solvates thereof, with the proviso that when R is hydrogen, R' is different from -(CH₂)ₙ-wherein n is 5 or 6.

Therefore, comprised within this primer aspect of the present invention there are included the salts of the compounds of general formula (I), particularly the pharmaceutically acceptable salts and the solvates of the compounds of general formula (I) and of the salts thereof, particularly of the pharmaceutically acceptable salts thereof.

The compounds of general formula (I), hereinafter compounds of the invention, have affinity for the histone deacetylase enzymes and are inhibitors thereof. They are useful therefore in the production of medicaments suitable for the treatment and/or prophylaxis of disorders or diseases sensitive to the inhibition of histone deacetylases. In the context of the present invention, disorders or diseases sensitive to the inhibition of histone deacetylases relate to the disorders or diseases in which the inhibition of histone deacetylases prevents the onset of said disorder or disease or achieves that a mammal's, including a human being's, health recovers or improves from a pathological condition. Proliferative type diseases such as cancer, particularly leukemia, solid and altered hormonal signal-dependent tumors and psoriasis, inflammatory type diseases, infection caused by HIV, Alzheimer's disease and senile dementia, among others, can be mentioned among said disorders or diseases.

In a particular embodiment of the compounds of the invention, R represents C1-C3 alkyl, phenyl, benzyl, F, Cl, Br, -OR1 wherein R1 represents hydrogen, C1-C3 alkyl, phenyl or benzyl, -N02, an amine of formula -NR1R2 wherein R1 and R2, the same or different, can be hydrogen, C1-C3 alkyl, phenyl, or benzyl, or an -NHCOR3 radical wherein R3 can be C1-C3 alkyl, phenyl or benzyl, and R' represents a -(CH₂)ₙ- group wherein n is 5 or 6.

In another particular embodiment of the compounds of the invention, R represents C1-C3 alkyl, F, Cl, Br, -OR1 wherein R1 is C1-C3 alkyl, phenyl or benzyl, -N02, NR1R2 wherein R1 is hydrogen and R2 represents a C1-C3 alkyl group, phenyl or benzyl, or -NHCOR3 wherein R3 is C1-C3 alkyl, phenyl or benzyl and R' represents a -(CH₂)ₙ- radical wherein n is 5 or 6.

In another particular embodiment of the compounds of the invention, R represents a methyl, ethyl, F, Cl, Br, -OCH₃, -OPh, -OBn, -N02, -NHR2 wherein R2 represents a C1-C3 alkyl group, phenyl or benzyl, or -NHCOCH₃, NHCOPh and R' represents a -(CH₂)ₙ- radical wherein n is 5 or 6.

In another particular embodiment of the compounds of the invention, R represents a methyl, ethyl or -N02 and R' represents a -(CH₂)ₙ- radical wherein n is 5 or 6 .

In another particular embodiment of the compounds of the invention, wherein R' represents a -(CH₂)ₙ- group wherein n is 5 or 6, R is located in position 5 of the phthalimide

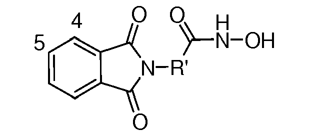

In another particular embodiment of the compounds of the invention, R represents hydrogen, C1-C3 alkyl, phenyl, benzyl, F, Cl, Br, -OR1 wherein R1 represents hydrogen, C1-C3 alkyl, phenyl or benzyl, -N02, an amine of formula - NR1 R2 wherein R1 and R2, the same or different, can be hydrogen, C1-C3 alkyl, phenyl, or benzyl, or an -NHCOR3 radical wherein R3 can be C1-C3 alkyl, phenyl or benzyl, and R' represents a 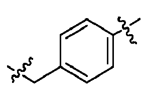 group.

In another particular embodiment of the compounds of the invention, R represents hydrogen, C1-C3 alkyl, F, Cl, Br, -OR1 wherein R1 is C1-C3 alkyl, phenyl or benzyl, -N02, NR1 R2 wherein R1 is hydrogen and R2 represents a C1-C3 alkyl group, phenyl or benzyl, or -NHCOR3 wherein R3 is C1-C3 alkyl, phenyl or benzyl and R' represents a 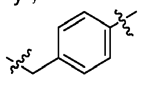 group.

In another particular embodiment of the compounds of the invention, R represents, hydrogen, methyl, ethyl, F, Cl, Br, -OCH₃, -OPh, -OBn, -N02, -NHR2 wherein R2 represents a C1-C3 alkyl group, phenyl or benzyl, or -NHCOCH₃, -NHCOPh and R' a 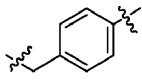 radical.

In another particular embodiment of the compounds of the present invention, wherein R' represents a 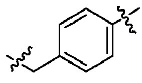 radical, the R substituent, different from hydrogen, is located in position 5 of the phthalimide

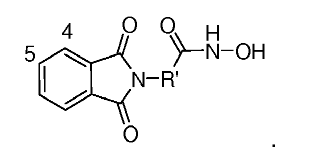

In another particular embodiment of the compounds of the present invention are selected from the following group:
[1 ] 6-(5-methyl-1,3-dioxoisoindol-2-yl)-N-hydroxyhexanamide (MTC-141)
[2] 6-(5-nitro-1,3-dioxoisoindol-2-yl)-N-hydroxyhexanamide (MTC-127)
[3] 4-[(1,3-dioxo-1,3-dihydro-isoindol-2-yl)-methyl]-N-hydroxybenzamide (MTC-126)

In another particular embodiment of the compounds of the present invention the R substituent different from H is located in position 4 of the phthalimide wherein R' represents a -(CH₂)ₙ- radical wherein n is 5 or 6 or a 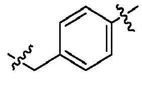 radical and said substituent is selected from among Cl, Br, F, C1-C3 alkyl, phenyl, benzyl, -OR1 wherein R1 is hydrogen, C1-C3 alkyl, phenyl, benzyl, -NO2, -NR1R2 wherein R1 and R2, the same or different, are selected independently from among hydrogen, C1-C3 alkyl, phenyl or benzyl, -NHCOR3 wherein R3 is C1-C3 alkyl, phenyl or benzyl.

In another additional aspect, the present invention provides a pharmaceutical composition comprising one or more compounds of general formula (I) or one of the pharmaceutically acceptable salts thereof, or one of the corresponding solvates thereof. The pharmaceutical composition of the present invention additionally comprises one or more pharmaceutically acceptable excipients for its administration, such as filler agents, solvents, diluents, coloring agents, coating agents, binders. The choice of the conventional excipients as well as the amount thereof depends on the route of administration intended and can easily be determined by the person skilled in the art. The pharmaceutical composition can be administered, among other routes, by rectal, parenteral, oral, buccal, topical, or inhalatory route. The pharmaceutical compositions provided by the present invention include, for example, tablets, sugar-coated tablets, capsules or multiparticulates such as pellets or granules, suitable solutions, suspensions or liquids, reconstitutable dry preparations, and also preparations for spraying. Likewise, said compositions can be delayed-release compositions generally known in the state of the art or can comprise an enteric coating.

Likewise, the present invention provides a compound of general formula (I) or one of the pharmaceutically acceptable salts thereof, or one of the corresponding solvates thereof for the treatment and/or prophylaxis of diseases sensitive to the inhibition of histone deacetylases in a mammal, including a human being.

In an additional aspect, the present invention provides a compound of general formula (I) or one of the pharmaceutically acceptable salts thereof, or one of the corresponding solvates thereof, for the treatment and/or prophylaxis of cancer, particularly leukemia, solid and altered hormonal signal-dependent tumors and psoriasis, inflammatory type diseases, infection caused by HIV, Alzheimer's disease and senile dementia in a mammal including a human being.

In another aspect, the present invention provides the use of a compound of general formula (I) or one of the pharmaceutically acceptable salts thereof, or one of the corresponding solvates thereof, in the production of a medicament for the treatment and/or prophylaxis of diseases sensitive to the inhibition of histone deacetylases. In an additional aspect, the present invention provides the use of a compound of general formula (I) or one of the pharmaceutically acceptable salts thereof, or one of the corresponding solvates thereof, in the production of a medicament for the treatment and/or prophylaxis of cancer, particularly leukemia, solid and altered hormonal signal-dependent tumors and psoriasis, inflammatory type diseases, infection caused by HIV, Alzheimer's disease and senile dementia in a mammal, including a human being.

In a final aspect, the present invention provides a process, which is shown in the following Scheme 1, for preparing a compound of general formula (I) 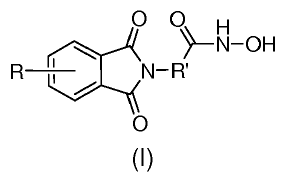 wherein
R' represents a -(CH₂)ₙ- radical wherein n is 5 or 6; and
R represents a C1-C3 alkyl, phenyl, benzyl, F, Cl, Br, -OR1 wherein R1 represents hydrogen, C1-C3 alkyl, phenyl or benzyl, -NO₂, an amine of formula -NR₁R₂ wherein R₁ and R2, the same or different, can be hydrogen, C1-C3 alkyl, phenyl, or benzyl, or an -NHCOR₃ radical wherein R₃ can be C1-C3 alkyl, phenyl or benzyl.

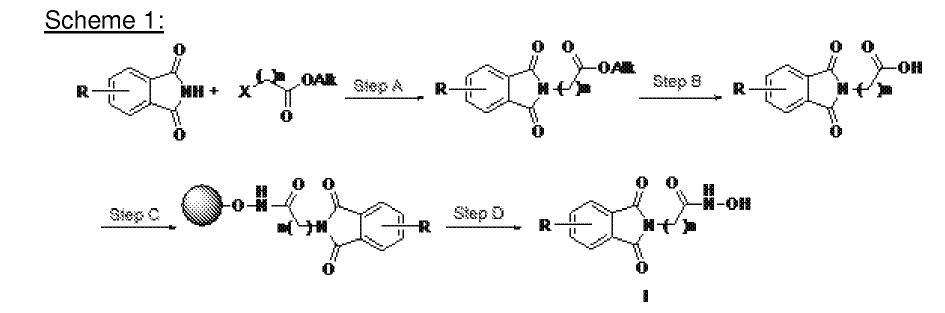

Said process comprises the following reaction steps A, B, C and D described below.

### Step A:

it comprises reacting a derivative of phthalimide, wherein R represents a C1-C3 alkyl, phenyl, benzyl, F, Cl, Br, -OR1 wherein R1 represents hydrogen, C1-C3 alkyl, phenyl or benzyl, -NO₂, an amine of formula -NR₁R₂ wherein R₁ and R2, the same or different, can be hydrogen, C1-C3 alkyl, phenyl, or benzyl, or an -NHCOR₃ radical wherein R₃ can be C1-C3 alkyl, phenyl or benzyl, with a compound of formula 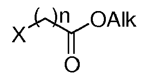 wherein
n is 5 or 6;
X represents a leaving group, such as for example, Br, Cl, -OSO₂CH₃ or a -OSO₂Ph(pCH₃); and
Alk represents an alkyl group such as for example an ethyl, methyl or t-butyl, or an acid function protecting group;
in the presence of an inorganic base and in an inert solvent.

### Step B:

it comprises the hydrolysis of the ester derivative obtained in step A of formula: 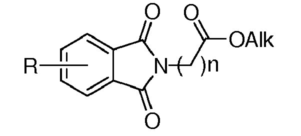 or alternatively, the elimination of the acid function protecting group, to obtain the corresponding carboxylic acid derivative of formula:

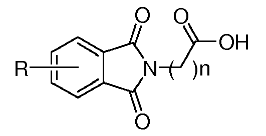

### Step C:

it comprises contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin of formula: 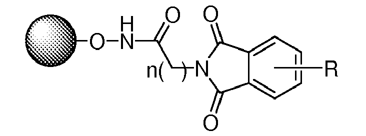 and

### Step D:

it comprises the release of the hydroxamic acid derivative bound to the Wang resin obtained in step C to obtain a compound of general formula (I) 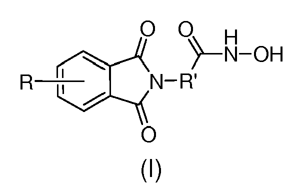 wherein R and R' have the previously defined meaning.

The invention also relates to another process, which is shown in the following Scheme 2, for preparing a compound of general formula (I) 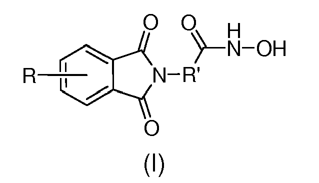 wherein R' is a 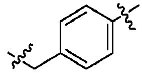 radical and
R represents hydrogen, C1-C3 alkyl, phenyl, benzyl, F, Cl, Br, -OR1 wherein R1 represents hydrogen, C1-C3 alkyl, phenyl or benzyl, -NO₂, an amine of formula -NR₁R₂ wherein R₁ and R2, the same or different, can be hydrogen, C1-C3 alkyl, phenyl, or benzyl or an -NHCOR₃ radical wherein R₃ can be C1-C3 alkyl, phenyl or benzyl.

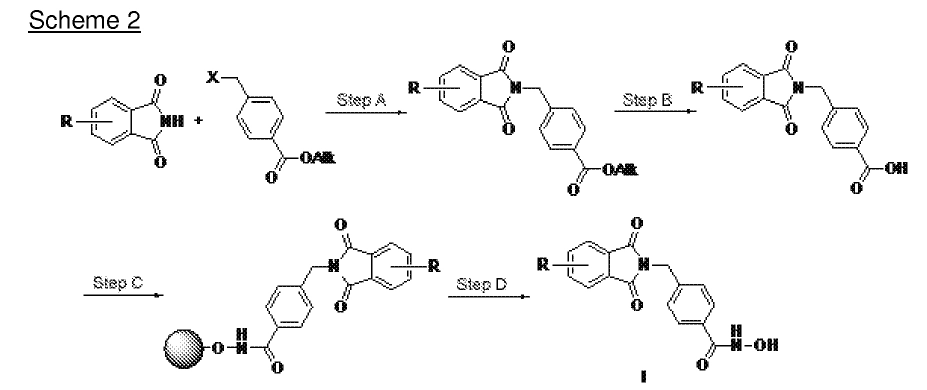

Said process comprises the following reaction steps A, B, C and D described below.

### Step A:

it comprises reacting a derivative of phthalimide, wherein R represents hydrogen, C1-C3 alkyl, phenyl, benzyl, F, Cl, Br, -OR1 wherein R1 represents hydrogen, C1-C3 alkyl, phenyl or benzyl, -NO₂, an amine of formula -NR₁R₂ wherein R₁ and R2, the same or different, can be hydrogen, C1-C3 alkyl, phenyl, or benzyl, or an -NHCOR₃ radical wherein R₃ can be C1-C3 alkyl, phenyl or benzyl, with a compound of formula: 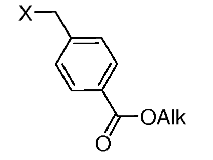 wherein
X represents a leaving group, such as for example, Br, Cl, -OSO₂CH₃ or a -OSO₂Ph(pCH₃); and
Alk represents an alkyl group such as for example ethyl, methyl or t-butyl, or an acid function protecting group;
in the presence of an inorganic base and in an inert solvent.

### Step B:

it comprises the hydrolysis of the ester derivative obtained in step A of formula: 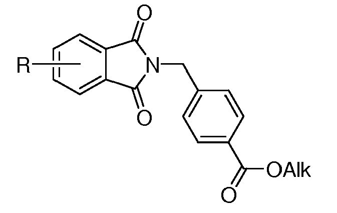 or alternatively, the elimination of the acid function protecting group to obtain the corresponding carboxylic acid derivative of formula:

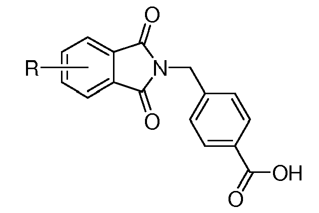

### Step C:

it comprises contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin of formula: 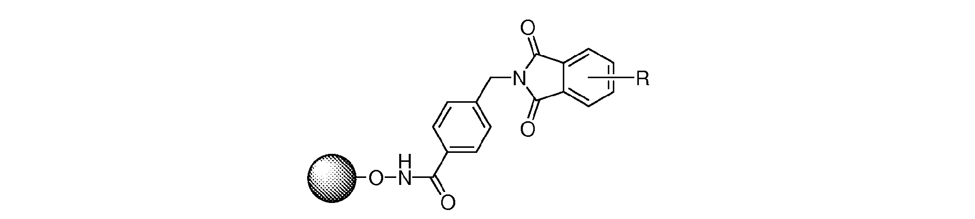 ; and

### Step D:

It comprises the release of the hydroxamic acid derivative bound to the Wang resin obtained in step C to obtain a compound of general formula (I) 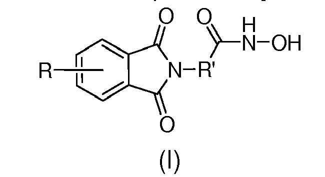 wherein R and R' have the previously defined meaning.

In both processes defined above for preparing the compounds of general formula (I) of the present invention, the reaction conditions of Steps A, B, C and D are common.

The starting compounds for step A: 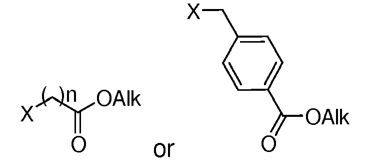 wherein X and Alk have the aforementioned meanings, are commercially available or can be easily prepared by a person skilled in the art by means of conventional processes.

The phthalimide and the starting derivatives R-substituted in 4 or 5, wherein R' represents a -(CH₂)ₙ- radical wherein n is 5 or 6 or a 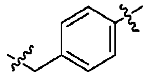 radical, can be obtained as described below. Some derivatives substituted in 4 or 5 of phthalimide, or phthalimide, are commercially available, as indicated below, other 4 or 5 substituted derivatives can be easily prepared by a person skilled in the art according to processes known in the state of the art and the synthesis of certain derivatives of phthalimides is performed according to the references indicated below.

In the particular case of the phthalimides substituted in position 5, the synthesis thereof is performed by means of the processes described below.

The 5 substituted phthalimide with R = Cl is commercially available (Nantong ChangChem, People's Republic of China). The 5 substituted phthalimide with R = Br is likewise commercially available (TCI EUROPE N.V., Belgium). The 5 substituted phthalimide with R = F is prepared as described in Watson, Timothy J.; Ayers, Timothy A.; Shah, Nik; Wenstrup, David; Webster, Mark; Freund, David; Horgan, Stephen; Carey, James P. Process Improvements for the Preparation of Kilo Quantities of a Series of Isoindoline Compounds. Organic Process Research & Development (2003), 7(4), 521-532.

The 5 substituted phthalimide with R = CH3 is commercially available (Sigma-Aldrich). The 5 substituted phthalimide with R = Et is commercially available (Chemstep). The preparation of the 5 substituted phthalimide with R = Pr is described in US 4427441. The 5 substituted phthalimide with R= Ph is commercially available (Aurora Fine Chemicals). The 5 substituted phthalimide with R = Bn is prepared from the anhydride with R = Bn in position 5, which is described in Gould, Ken J.; Hacker, Nigel P.; McOmie, John F. W.; Perry, David H. Benzocyclobutenes. Part 4. Synthesis of benzocyclobutene-1,2-diones by pyrolytic methods. Journal of the Chemical Society, Perkin Transactions 1: Organic and Bio-Organic Chemistry (1972-1999) (1980), (8), 1834-40, and following the methodology described in Peng, Yanqing; Song, Gonghua; Qian, Xuhong. Imidation of cyclic carboxylic anhydrides under microwave irradiation. Synthetic Communications (2001), 31(12), 1927-1931.

The 5 substituted phthalimide with R = OH or with R = OBn are prepared as described in Gnerre, Carmela; Catto, Marco; Leonetti, Francesco; Weber, Peter; Carrupt, Pierre-Alain; Altomare, Cosimo; Carotti, Angelo; Testa, Bernard. Inhibition of Monoamine Oxidases by Functionalized Coumarin Derivatives: Biological Activities, QSARs, and 3D-QSARs. Journal of Medicinal Chemistry (2000), 43(25), 4747-4758. The 5 substituted phthalimide with R = OPh is commercially available (Aurora Fine Chemicals). The 5 substituted phthalimide with R = OMe is obtained as described in Yoon, Ung Chan; Kim, Dong Uk; Lee, Chan Woo; Choi, Young Sun; Lee, Yean-Jang; Ammon, Herman L.; Mariano, Patrick S. Novel and Efficient Azomethine Ylide Forming Photoreactions of N-(Silylmethyl)phthalimides and Related Acid and Alcohol Derivatives. Journal of the American Chemical Society (1995), 117(10), 2698-710; the 5 substituted phthalimide with R = OEt is obtained as described in US 4207112; and the 5 substituted phthalimide with R = OPr is obtained as described in Brown, Frank K.; Brown, Peter J.; Bickett, D. Mark; Chambers, C. Lynn; Davies, H. Geoff; Deaton, David N.; Drewry, David; Foley, Michael; McElroy, Andrew B.; et al. Matrix Metalloproteinase Inhibitors Containing a [(Carboxyalkyl)amino]zinc Ligand: Modification of the P1 and P2' Residues. Journal of Medicinal Chemistry (1994), 37(5), 674-88.

The phthalimide with R = N02 in position 5 is commercially available (Sigma-Aldrich), and the phthalimide with R = NH2 in position 5 is likewise commercially available (Acros Organics).

The 5 substituted phthalimides with R = -NR1 R2 are generally prepared following the processes described in [a) Lee, D.; Hartwig, J. F. Zinc trimethylsilylamide as a mild ammonia equivalent and base for the amination of aryl halides and triflates. Organic Letters 2005, 7(6), 1169-1172; b) Gajare, A. S.; Toyota, K.; Yoshifuji, M.; Ozawa, F. Solvent free amination reactions of aryl bromides at room temperature catalyzed by a (π-allyl)palladium complex bearing a diphosphinidenecyclobutene ligand. Journal of Organic Chemistry 2004, 69(19), 6504-6506; or c) Rao, H; Fu, H.; Jiang, Y.; Zhao, Y. Copper-Catalyzed Arylation of Amines Using Diphenyl Pyrrolidine-2-phosphonate as the New Ligand. Journal of Organic Chemistry 2005, 70(20), 8107-8109.] which is prepared starting from 5 substituted phthalimide with R = Br and is subjected to the reaction conditions described in the previous references in the presence of R1 R2NH wherein R1 and R2 have the previously mentioned values.

The starting phthalimide wherein R in position 5 is -NHCOR3, and R3 represents C1-C3 alkyl, phenyl or benzyl can be prepared from the phthalimide -NH2 substituted in position 5, before performing step A of the process of the present invention, by reacting with the corresponding acid chloride derivative Cl-CO-R3 in a conventional manner. Some of these phthalimides are particularly likewise commercially available.

In the particular case of the phthalimides substituted in position 4 the synthesis of the following derivatives is performed by means of the processes described below.

The starting phthalimide wherein R is Cl can be prepared as described in Clark, Robin D.; Berger, Jacob; Garg, Pushkal; Weinhardt, Klaus K.; Spedding, Michael; Kilpatrick, Andrew T.; Brown, Christine M.; MacKinnon, Alison C. Affinity of 2-(tetrahydroisoquinolin-2-ylmethyl)- and 2-(isoindolin-2-ylmethyl)imidezolines for π -adrenoceptors. Differential affinity of imidezolines for the [3H]idazoxan-labeled π 2-adrenoceptor vs. the [3H]yohimbine-labeled site. Journal of Medicinal Chemistry (1990), 33(2), 596-600. It can alternatively be prepared from the commercially available anhydride substituted with Cl in position 4 (Acros Organics) which is treated according to the conditions described in Peng, Yanqing; Song, Gonghua; Qian, Xuhong. Imidation of cyclic carboxylic anhydrides under microwave irradiation. Synthetic Communications (2001), 31(12), 1927-1931.

The starting phthalimide wherein R is Br can be prepared as described in Rabjohn, Norman; Drumm, M. F.; Elliott, R. L. Some reactions of N-acetylphthalimides. Journal of the American Chemical Society (1956), 78 1631-4. It is alternatively prepared from the commercially available anhydride substituted with Br in position 4 (DSL Chemicals, Shanghai) which is treated in the conditions described in Peng, Yanqing; Song, Gonghua; Qian, Xuhong. Imidation of cyclic carboxylic anhydrides under microwave irradiation. Synthetic Communications (2001), 31 (12), 1927-1931.

The starting phthalimide wherein R is F can be prepared as described in DE 3320089. It is alternatively prepared from the corresponding commercially available anhydride substituted in position 4 with fluorine (Sigma-Aldrich) which is treated in the conditions described in Peng, Yanqing; Song, Gonghua; Qian, Xuhong. Imidation of cyclic carboxylic anhydrides under microwave irradiation. Synthetic Communications (2001), 31(12), 1927-1931.

The starting phthalimide wherein R is methyl is commercially available (Aurora Fine Chemicals). The starting phthalimide wherein R is ethyl can be prepared from the anhydride (commercially available) as described in Woods, G. F.; Bolgiano, N. C.; Duggan, D. E. The chemistry of 1,3,5-hexatriene. Journal of the American Chemical Society (1955), 77, 1800-3. It is alternatively prepared from the anhydride with R=Et in position 4 which is treated in the conditions described in the following reference Peng, Yanqing; Song, Gonghua; Qian, Xuhong. Imidation of cyclic carboxylic anhydrides under microwave irradiation. Synthetic Communications (2001), 31 (12), 1927-1931. The starting phthalimide wherein R is propyl can be prepared from the anhydride with R =Pr in position 4 described in Fleischhacker, Herman; Woods, G. Forrest. Methyl-1,3,5-hexatrienes. Journal of the American Chemical Society (1956), 78, 3436-9, which is treated according to the conditions described in (Peng, Yanqing; Song, Gonghua; Qian, Xuhong. Imidation of cyclic carboxylic anhydrides under microwave irradiation. Synthetic Communications (2001), 31(12), 1927-1931.). The starting phthalimide wherein R is Ph can be prepared from the anhydride with R=Ph in position 4 described in [a) Atkinson, C. M.; Sharpe, C. J. Synthesis of some phenylcinnolines, -phthalazines, and -quinoxalines. Journal of the Chemical Society (1959), 2858-64; b) Bestmann, H. J.; Kloeters, W. The reaction of hexaphenylcarbodiphosphorane with cyclic aromatic carboxylic acid anhydrides. Tetrahedron Letters (1978), (36), 3343-4], which is treated in the conditions described in Peng, Yanqing; Song, Gonghua; Qian, Xuhong. Imidation of cyclic carboxylic anhydrides under microwave irradiation. Synthetic Communications (2001), 31(12), 1927-1931. The starting phthalimide wherein R is Bn is prepared from the anhydride with R = Bn, described in Mavoungou-Gomes, Louis. Naphtho[2,3-b]furans. Comptes Rendus des Seances de l'Academie des Sciences, Serie C: Sciences Chimiques (1970), 270(8), 750-3, which is treated in the conditions described in the following reference to obtain the phthalimide with R = Bn (Peng, Yanqing; Song, Gonghua; Qian, Xuhong. Imidation of cyclic carboxylic anhydrides under microwave irradiation. Synthetic Communications (2001), 31 (12), 1927-1931.).

The starting phthalimide wherein R is -OH can be prepared as described in the reference Muller, George W.; Corral, Laura G.; Shire, Mary G.; Wang, Hua; Moreira, Andre; Kaplan, Gilla; Stirling, David I. Structural Modifications of Thalidomide Produce Analogs with Enhanced Tumor Necrosis Factor Inhibitory Activity. Journal of Medicinal Chemistry (1996), 39(17), 3238-3240. The starting phthalimide wherein R is -OBn is prepared from the anhydride with R = OBn, described in the reference Nagasaka, Tatsuo; Koseki, Yuji. Stereoselective Synthesis of Tilivalline. Journal of Organic Chemistry (1998), 63(20), 6797-6801, which is treated in the conditions described in the following reference to obtain the phthalimide with R = OBn (Peng, Yanqing; Song, Gonghua; Qian, Xuhong. Imidation of cyclic carboxylic anhydrides under microwave irradiation. Synthetic Communications (2001), 31 (12), 1927-1931.). The starting phthalimide wherein R is -OPh can be prepared from the anhydride with R = OPh, described in the reference Williams, F. J.; Relles, H. M.; Donahue, P. E.; Manello, J. S. A direct synthesis of phenoxy-substituted phthalic anhydrides by aromatic nucleophilic displacement. Journal of Organic Chemistry (1977), 42(21), 3425-31, which is treated in the conditions described in the following reference to obtain the phthalimide with R = Ph (Peng, Yanqing; Song, Gonghua; Qian, Xuhong. Imidation of cyclic carboxylic anhydrides under microwave irradiation. Synthetic Communications (2001), 31 (12), 1927-1931.). The starting phthalimide wherein R is -OMe can be prepared as described in the references [a) Watanabe, Tokuhiro; Hamaguchi, Fumiko; Ohki, Sadao. Reduction of cyclic imides. III. Reduction of 3- and 4-substituted phthalimides with sodium borohydride. Chemical & Pharmaceutical Bulletin (1978), 26(2), 530-8; b) Bentley, W. H.; Robinson, R.; Weizmann, C. 3-Hydroxyphthalic and 3-Methoxyphthalic Acids and Their Derivatives. Journal of the Chemical Society, Transactions (1907), 91 104-12.] The starting phthalimide wherein R is -OEt can be prepared from the anhydride with R = OEt, described in the reference Breau, Livain; Kayser, Margaret M. On the regioselectivity of the condensation of stabilized phosphorus ylides with 3-substituted phthalic anhydrides. Canadian Journal of Chemistry (1989), 67(4), 569-73, which is treated in the conditions described in the following reference to obtain the phthalimide with R = OEt (Peng, Yanqing; Song, Gonghua; Qian, Xuhong. Imidation of cyclic carboxylic anhydrides under microwave irradiation. Synthetic Communications (2001), 31(12), 1927-1931). The starting phthalimide wherein R is -OPr can be prepared from the anhydride with R = OPr, described in the reference Da Settimo, Antonio; Primofiore, Giampaolo; Ferrarini, Pier Luigi; Livi, Oreste; Tellini, Natale; Bianchini, Pietro. Synthesis and local anesthetic activity of some N-β-diethylaminoethylphthalimides. European Journal of Medicinal Chemistry (1981), 16(1), 59-64, which is treated in the conditions described in the following reference to obtain the phthalimide with R = OPr (Peng, Yanqing; Song, Gonghua; Qian, Xuhong. Imidation of cyclic carboxylic anhydrides under microwave irradiation. Synthetic Communications (2001), 31(12), 1927-1931).

The starting phthalimides wherein R is N02 or R is -NH2 are commercially available (Sigma-Aldrich)

The starting phthalimides wherein R is -NR1 R2 can be prepared as described below. The phthalimide substituted in position 4 with Br is used to start and it is subjected to the reaction conditions described in the following references in the presence of a desired amine of formula R1 R2NH wherein R1 and R2 have the previously described meanings (Lee, D.; Hartwig, J. F. Zinc trimethylsilylamide as a mild ammonia equivalent and base for the amination of aryl halides and triflates. Organic Letters 2005, 7(6), 1169-1172); (Gajare, A. S.; Toyota, K.; Yoshifuji, M.; Ozawa, F. Solvent free amination reactions of aryl bromides at room temperature catalyzed by a (π-allyl)palladium complex bearing a diphosphinidenecyclobutene ligand. Journal of Organic Chemistry 2004, 69(19), 6504-6506.); (Rao, H; Fu, H.; Jiang, Y.; Zhao, Y. Copper-Catalyzed Arylation of Amines Using Diphenyl Pyrrolidine-2-phosphonate as the New Ligand. Journal of Organic Chemistry 2005, 70(20), 8107-8109.).

The starting phthalimide wherein R is -NHCOR3, and R3 represents C1-C3 alkyl, phenyl or benzyl can be prepared from the phthalimide -NH2 substituted in position 4, before performing step A, by reacting with the corresponding acid chloride derivative Cl-CO-R3 in a conventional manner.

With respect to the starting compounds of general formulas: 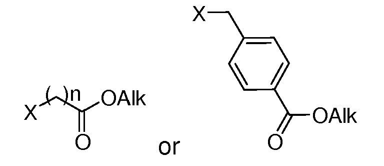 Alk, as previously mentioned, can be an acid function protecting group. Said protecting group can be any conventional protecting group known by a person skilled in the art, such as for example an allyl protecting group to form allyl esters. It can be deprotected by means of conventional methods, for example, in the presence of palladium (0), triphenylphosphine and phenylsilane. Depending on the nature thereof, the conditions for deprotection in step B of the process are also known by a person skilled in the art.

Step A of the previously described processes is performed in the presence of an inorganic base and in an inert solvent. In a particular embodiment said inorganic base is a carbonate, such as for example potassium carbonate. In a particular embodiment said inert solvent is dimethylformamide (DMF). The starting materials are reacted by heating the reaction mixture at a suitable temperature depending on the solvent, typically around 120ºC and for a time which may vary depending on the starting materials, and reaction conditions, which is typically 24 hours. The intermediate reaction obtained product in step A can be precipitated by adding water-ice, is filtered, and is obtained in the form of a solid which can be vacuum-dried. The intermediate reaction products obtained in step A which do not precipitate in the previous conditions can alternatively be extracted with a suitable organic solvent, such as for example ethyl acetate. The organic phase is dried, filtered and concentrated in the rotary evaporator. The obtained product can optionally be purified by means of flash chromatography using mixtures of suitable solvents such as for example ethyl acetate/hexane.

Hydrolysis of the product obtained in step A is performed in step B. In the event that said derivative is an ester derivative, the hydrolysis thereof is typically performed in the presence of an acid by heating. In a particular embodiment concentrated hydrochloric acid is used. The obtained intermediate product is precipitated by adding water, filtered and optionally purified by means of flash chromatography using mixtures of suitable solvents, such as for example dichloromethane/methanol.

In step C, the Wang resin functionalized with hydroxylamine is a commercially available product. It is conditioned with a polar aprotic inert solvent, typically dichloromethane. It is then filtered and washed with an inert solvent, typically dimethylformamide. HOAt, DIPCDI and the carboxylic acid derivative obtained in step B in a suitable solvent, particularly DMF, is added. Once the reaction between the carboxylic acid derivative and the functionalized resin has ended, it is filtered, typically washed with DMF and conditioned with dichloromethane.

In step D the hydroxamic acid derivative bound to the resin is released by adding an acid in a solvent. In a particular embodiment TFA in DCM is used. The resulting derivative of general formula (I) can be purified and/or isolated according to conventional processes known by persons skilled in the art. In a particular embodiment this is done by means of flash chromatography and is characterized by NMR (Nuclear Magnetic Resonance) and MS (mass spectrometry).

During one or more steps of the synthesis processes described above and represented in Schemes 1 and 2, or in the preparation of the starting phthalimides, it may be necessary and/or desirable to protect sensitive or reactive groups in some of the molecules used. This can be done by means of conventional protecting groups such as those described in the literature [a) T. W. Greene & P. G. M. Wuts, Protective Groups in Organic Chemistry, John Wiley & Sons, 3rd edition, 1999; b) Philip J. Kocienski, Protecting Groups, Thieme, 3rd edition, 2004]. The protecting groups can be removed in a suitable subsequent step by processes known by persons skilled in the art. The respective descriptions in the literature are incorporated in the specification as a reference and form parte of the description. In a particular embodiment when the starting phthalimide of step A is - NH2 substituted in position 4 or 5, said group can, for example, be protected with the protecting group Fmoc (9-fluorenylmethoxycarbonyl) as described in the literature. The deprotection is performed in a conventional manner according to the conditions described in the literature before performing step D of releasing the compound from the Wang resin.

The pharmacologically acceptable salts of the compounds of general formula (I) can be prepared by conventional processes known by persons skilled in the art, comprising reacting with a base to form the corresponding addition salt, for example, ammonium, alkali, or alkali-earth salts, particularly of lithium, sodium, potassium, magnesium, calcium, or a salt with an organic base such as benzathine, N-methyl-D-glucamine, or with amino acids such as lysine or arginine.

The physiologically acceptable solvates, particularly hydrates and alcoholates of the derivatives of general formula (I) or of the corresponding physiologically acceptable salts thereof, can be prepared by conventional processes known by persons skilled in the art.

### EXAMPLES

### Example 1. Synthesis of 6-(5-methyl-1,3-dioxoisoindol-2-yl)-N-hydroxyhexanamide (MTC-141)

The synthesis comprised the following steps:

### 1.1 Synthesis of ethyl 6-(5-methyl-1,3-dioxo-isoindol-2-yl)hexanoate

The starting materials were 5-methyl-phthalimide (300 mg, 1.86 mmol) and K₂CO₃ (257.39 mg, 1.86 mmol), in 2 mL of DMF. 6-bromoethyl hexanoate (0.33 mL, 1.86 mmol) was added to this solution, and the reaction mixture was heated at 120ºC for 24 hours. After the reaction time elapsed, it is diluted with water and an extraction with ethyl acetate (3x5mL) was performed, the organic phase was dried on MgSO₄, filtered and concentrated in the rotary evaporator. A yellow liquid was obtained (564 mg, quantitative yield).
¹H-NMR (C₃D₆O): δ 7.70 (d, 1 H, Hₐᵣₒₘ, J = 7.8 Hz); 7.61 (d, 2H, Hₐᵣₒₘ, J = 8.4 Hz ); 4.03 (c, 2H, O-CH₂CH₃, J = 7.1 Hz); 3.61 (t, 2H, N-CH₂, J = 7.1 Hz); 2.50 (s, 3H, Ar-CH₃); 2.26 (t, 2H, CH₂-CO, J = 7.4 Hz); 1.70 - 1.57 (m, 4H, CH₂); 1.40 - 1.32 (m, 2H, CH₂); 1.16 (t, 3H, O-CH₂CH₃, *J* = 7.2 Hz).
¹³C₋NMR (C₃D₆O): 172.7 (COOEt); 168.1 (CO); 168.0 (CO); 145.4 (Cₐᵣₒₘ); 134.5 (CHₐᵣₒₘ); 132.8 (Cₐᵣₒₘ); 129.8 (Cₐᵣₒₘ); 123.4 (CHₐᵣₒₘ); 122.8 (CHₐᵣₒₘ); 59.6 (O-CH₂CH₃); 37.4 (N-CH₂); 33.6 (CH₂-CO); 28.1 (CH₂); 26.1 (CH₂); 24.4 (CH₂); 21.0 (Ar-CH₃); 13.7 (O-CH₂CH₃).
HR LSIMS: Calculated for C₁₇H₂₁NO₄Na (M+Na)⁺ 326.1368; found 326.1371 (deviation -0.9 ppm).

### 1.2 Synthesis of the 6-(5-methyl-1,3-dioxo-isoindol-2-yl)hexanoic acid

The compound obtained in the previous step 6-(5-methyl-1,3-dioxo-isoindol-2-yl) ethyl hexanoate (500 mg, 1.85 mmol) and 4.5 mL of concentrated HCl were used as the starting material; the reaction mixture was heated under reflux for 24 hours. The reaction product obtained was precipitated after adding water and was filtered. It was purified by means of flash chromatography using dichloromethane/methanol as an eluent. A white solid (371 mg) with m.p. = 110-112ºC (73% yield) was obtained.
¹H-NMR (C₃D₆O): δ 10.40 (bs, 1 H, COOH); 7.70 (d, 1 H, Hₐᵣₒₘ, *J*= 7.7 Hz); 7.61 (d, 1 H, Hₐᵣₒₘ, *J* = 8.4 Hz); 3.61 (t, 2H, N-CH₂, *J* = 7.2 Hz); 2.50 (s, 3H, CH₃); 2.28 (t, 2H, CH₂-CO, *J* = 7.4 Hz); 1.71 - 1.57 (m, 4H, CH₂); 1.42 - 1.31 (m, 2H, CH₂).
¹³C-NMR (C₃D₆O): 174.5 (COOH); 168.9 (CO); 168.8 (CO); 146.2 (Cₐᵣₒₘ); 135.3 (CHₐᵣₒₘ); 133.5 (Cₐᵣₒₘ); 130.6 (Cₐᵣₒₘ); 124.1 (CHₐᵣₒₘ); 123.6 (CHₐᵣₒₘ); 38.2 (N-CH₂); 34.0 (CH₂-CO); 29.0 (CH₂); 27.0 (CH₂); 25.2 (CH₂); 21.7 (CH₃).
HR LSIMS: Calculated for C₁₅H₁₇NO₄Na (M+Na)⁺ 298.1055; found 298.1057 (deviation -0.5 ppm).

### 1.3 Synthesis of 6-(5-methyl-1,3-dioxoisoindol-2-yl)-N-hydroxyhexanamide (MTC-141)

A Wang resin functionalized with hydroxylamine (0.2 mmol) was conditioned in dichloromethane (DCM) for 6 hours. It was then washed with dimethylformamide (DMF, 2 x 4 mL) and a solution of hydroxyazabenzotriazole (HOAt, 0.8 mmol), diisopropylcarbodiimide (DIPCDI, 0.8 mmol) and 6-(5-methyl-1,3-dioxo-isoindol-2-yl)hexanoic acid (238 mg, 0.8 mmol) in DMF was added. After 24 hours of reaction, the resin was washed with DMF (3x4mL), and was conditioned with DCM (3x4mL). The hydroxamic acid derivative bound to the resin was released from the resin by means of adding 10 mL of a solution of trifluoroacetic acid (TFA) in DCM at 50% for 30 minutes. Finally, the resin was washed with DCM and the organic phases were concentrated in the rotary evaporator. The end product was purified by flash chromatography using DCM:MeOH (10:0.3) as an eluent. A white solid was obtained (20 mg, yield = 39%).
¹H-NMR (DMSO-d₆): δ 10.28 (s, 1 H, NHOH); 8.62 (s, 2H, OH); 7.73 - 7.59 (m, 3H, Hₐᵣₒₘ); 3.50 (t, 2H, N-CH₂, J = 7.3 Hz); 2.45 (s, 3H, CH₃); 1.89 (t, 2H, CH₂-CO, *J* = 7.4 Hz); 1.58 - 1.42 (m, 4H, CH₂); 1.24 - 1.14 (m, 2H, CH₂).
¹³C-NMR (DMSO-d₆): 168.8 (CONHOH); 167.9 (CO); 167.8 (CO); 145.2 (Cₐᵣₒₘ); 134.5 (CHₐᵣₒₘ); 131.8 (Cₐᵣₒₘ); 128.8 (Cₐᵣₒₘ); 123.3 (CHₐᵣₒₘ); 122.8 (CHₐᵣₒₘ); 37.1 (N-CH₂); 31.9 (CH₂-CO); 27.6 (CH₂); 25.7 (CH₂); 24.5 (CH₂); 21.2 (CH₃).
HR LSIMS: Calculated for C₁₅H₁₈N₂O₄Na (M+Na)⁺ 313.1164; found 313.1160 (deviation 1.4 ppm).

### Example 2. Synthesis of the inhibitor 6-(5-nitro-1,3-dioxo-isoindol-2-yl)-N-hydroxyhexanamide (MTC-127)

The synthesis comprised the following steps:

### 2.1 Synthesis of ethyl 6-(5-nitro-1,3-dioxo-isoindol-2-yl)hexanoate

The starting materials were 5-nitro-phthalimide (300 mg, 1.56 mmol) and K₂CO₃ (216 mg, 1.56 mmol), in 2 mL of DMF. 6-bromoethyl hexanoate (0.28 mL, 1.56 mmol) was added to this solution and the reaction mixture was heated at 120ºC for 24 hours. After the reaction time elapsed, an extraction with ethyl acetate (3x5mL) was performed, the organic phase was dried on MgSO₄, filtered and concentrated in the rotary evaporator. The obtained product was used in the following reaction step without more purification.

### 2.2 Synthesis of 6-(5-nitro-1,3-dioxo-isoindol-2-yl)hexanoic acid

The starting materials were 6-(5-nitro-1,3-dioxo-isoindol-2-yl) ethyl hexanoate (500 mg, 1.5 mmol) and 4.5 mL of concentrated HCl and the mixture was heated under reflux for 24 hours. The obtained product was used in the next reaction without more purification.

### 2.3 Synthesis of 6-(5-nitro-1,3-dioxo-isoindol-2-yl)-N-hydroxyhexanamide (MTC-127)

The Wang resin functionalized with hydroxylamine (0.2 mmol) (Novabiochem) was conditioned in DCM for 6 hours. The resin was filtered, the washed with DMF (2x4mL), and a solution of hydroxyazabenzotriazole (HOAt, 0.8 mmol), diisopropylcarbodiimide (DIPCDI, 0.8 mmol) and 6-(5-nitro-1,3-dioxo-isoindol-2-yl)hexanoic acid (245 mg, 0.8 mmol) in DMF was added. After 24 hours of reaction, the resin was filtered, washed with DMF (3x4mL), and conditioned with DCM (3x4mL). The hydroxamic acid derivative bound to the resin was released by adding 10 mL of a solution of trifluoroacetic acid (TFA) in DCM at 50% for 30 minutes. After this time, the resin was washed with DCM and the organic phases were concentrated in the rotary evaporator. The end product was recrystallized from a mixture of DCM/hexane. A white solid with m.p. = 130-132ºC was obtained. ¹H-NMR (DMSO-d₆): δ 10.28 (s, 1 H, NHOH); 8.60 (s, 1 H, OH); 8.59 (dd, 2H, Hₐᵣₒₘ, *J* = 1.9 Hz, *J* = 8.1 Hz ); 8.45 (d, 1 H, Hₐᵣₒₘ, *J* = 1.8 Hz); 8.09 (d, 1 H, Hₐᵣₒₘ, *J* = 8.1 Hz); 3.57 (t, 2H, N-CH₂, *J* = 7.0 Hz); 1.90 (t, 2H, CH₂-CO, *J* = 7.3 Hz); 1.62 - 1.43 (m, 4H, CH₂); 1.28-1.23 (m, 2H, CH₂).
¹³C-NMR (DMSO-d₆): 168.8 (CONHOH); 166.2 (CO); 165.9 (CO); 151.2 (NO₂-Cₐᵣₒₘ); 136.2 (Cₐᵣₒₘ); 132.9 (Cₐᵣₒₘ); 129.4 (CHₐᵣₒₘ); 124.3 (CHₐᵣₒₘ); 117.6 (CHₐᵣₒₘ); 37.8 (N-CH₂); 31.9 (CH₂-CO); 27.4 (CH₂); 25.7 (CH₂); 24.5 (CH₂).
HR LSIMS: Calculated for C₁₄H₁₅N₃O₆Na (M+Na)⁺ 344.0859; found 344.0856 (deviation 0.7 ppm).

### Example 3. Synthesis of 4-[(1,3-dioxo-1,3-dihydro-isoindol-2-yl)-methyl]-N-hydroxybenzamide (MTC-126):

The synthesis comprised the following steps:

### 3.1 Synthesis of ethyl 4-[(1,3-dioxo-1,3-dihydro-isoindol-2-yl)methyl]benzoate

The starting materials were phthalimide (300 mg, 2.04 mmol) and K₂CO₃ (282 mg, 2.04 mmol) in 2 mL of DMF. 6-bromoethyl hexanoate (0.36 mL, 2.04 mmol) was added to this solution, and the reaction mixture was heated at 120ºC for 24 hours. After the reaction time elapsed, an extraction with ethyl acetate (3x5mL) was performed, the organic phase was dried on MgSO₄, filtered and concentrated in the rotary evaporator. The obtained product was used in the next reaction without more purification.

### 3.2 Synthesis of 4-[(1,3-dioxo-1,3-dihydro-isoindol-2-yl)methyl]benzoic acid

The starting materials were 4-[(1,3-dioxo-1,3-dihydro-isoindol-2-yl)methyl] ethyl benzoate (500 mg, 1.62 mmol) and 4.5 mL of concentrated HCl and the mixture was heated under reflux for 24 hours. The precipitate formed was purified by flash chromatography using DCM:MeOH (10:1) as an eluent. A white solid with m.p. = 258-259ºC was obtained.
¹H-NMR (DMSO-d₆): δ 7.90 - 7.82 (m, 6H, Hₐᵣₒₘ); 7.38 (d, 2H, Hₐᵣₒₘ, *J* = 8.3 Hz); 4.81 (s, 2H, CH₂).
¹³C-NMR (DMSO-d₆): 168.2 (CO); 167.8 (COOH); 141.8 (Cₐᵣₒₘ); 135.1 (CHₐᵣₒₘ); 132.1 (Cₐᵣₒₘ); 131.0 (Cₐᵣₒₘ); 130.1 (CHₐᵣₒₘ); 127.9 (CHₐᵣₒₘ); 123.8 (CHₐᵣₒₘ); 41.2 (CH₂).
HR LSIMS: Calculated for C₁₆H₁₁NO₄Na (M+Na)⁺ 304.0586; found 304.0583 (deviation 1.0 ppm).

### 3.3 Synthesis of 4-[(1,3-dioxo-1,3-dihydro-isoindol-2-yl)-methyl]-N-hydroxybenzamide (MTC-126)

The Wang resin functionalized with hydroxylamine (0.2 mmol) (Novabiochem) was conditioned in DCM for 6 hours. The resin was filtered, then washed with DMF (2x4mL), and a solution of hydroxyazabenzotriazole (HOAt, 0.8 mmol), diisopropylcarbodiimide (DIPCDI, 0.8 mmol) and 4-[(1,3-dioxo-1,3-dihydro-isoindol-2-yl)methyl]benzoic acid (152 mg, 0.54 mmol) in DMF was added. After 24 hours of reaction, the resin was filtered, washed with DMF (3x4mL), and conditioned with DCM (3x4mL). The hydroxamic acid derivative bound to the resin was released by adding 10 mL of a solution of trifluoroacetic acid (TFA) in DCM at 50% for 30 minutes. After this time, the resin was washed with DCM and the organic phases were concentrated in the rotary evaporator.

The end product obtained was purified by flash chromatography using DCM:MeOH (10:0.3) as an eluent. A white solid (32 mg) with m.p. = 190-192ºC (yield = 60%) was obtained.
¹H-NMR (DMSO-d₆): δ 11.15 (s, 1 H, NHOH); 8.98 (s, 1 H, OH); 7.90 - 7.80 (m, 4H, Hₐᵣₒₘ); 7.67 (d, 2H, Hₐᵣₒₘ, *J* = 8.2 Hz); 7.34 (d, 2H, Hₐᵣₒₘ, *J* = 8.3 Hz); 4.79 (s, 2H, CH₂).
¹³C-NMR (DMSO-d₆): 167.5 (CONHOH); 163.8 (CO); 139.5 (Cₐᵣₒₘ); 134.4 (CHₐᵣₒₘ); 131.8 (Cₐᵣₒₘ), 131.4 (Cₐᵣₒₘ); 127.1 (CHₐᵣₒₘ); 123.1 (CHₐᵣₒₘ); 40.4 (CH₂).

### Example 4

The inhibitory activity of the compounds obtained in Examples 1, 2 and 3 above was evaluated, for which the HDAC AK-501 colorimetric titration kit for inhibition, supplied by Biomol, was used, following the indicated protocol.

The process of the assay was carried out in two steps. In the first step, the Color de Lys^{®} substrate, containing an acetylated lysine group, was incubated with a sample of HeLa (human cervical cancer cell line) nuclear extract, rich in HDAC activity. In the second step, the previous mixture was treated with the Color de Lys^{®} developer, which caused an increase in the quantifiable color intensity at 405 nm. There is a linear correlation between the absorption and the deacetylation of lysine within instrumental limits.

The operating procedure was the following:
1. The buffer, diluted (250 nM) TSA (Trichostatin A) and the inhibitor to be assayed were added at the desired concentrations in the suitable wells of the plate (See Table below).
2. The Hela extract was added, except to the enzyme-free control.
3. The plate and the Color de Lys^{®} substrate were thermostatted at 37ºC.
4. The reactions were started adding the 0.4 mM substrate (final concentration of the substrate 0.2 mM) in each well and it was stirred.
5. A reaction time of 20 minutes at 37ºC was allowed.
6. The Color de Lys^{®} developer, prepared approximately 30 minutes before its use, was added (the developer was used diluted 20 times with an amount of TSA resulting in a final concentration of 1µM in the assay). It was allowed to react for 15 minutes at 37 ºC.
7. The reading was made at 405 nm.

**Table**

| **Wells** | **Butter Buffer** | **HeLa** | **Inhibitor (x5)** | **Substrate (x2)** | **Developer** |
|---|---|---|---|---|---|
| **Target** | 25 µL | 0 | 0 | 25 µL | 50 µL |
| **Control** | 20 µL | 5 µL | 0 | 25 µL | 50 µL |
| **TSA** | 10 µL | 5 µL | 10 µL | 25 µL | 50 µL |
| **Inhibitor** | 10 µL | 5 µL | 10 µL | 25 µL | 50 µL |

| | | | | | |
|---|---|---|---|---|---|
| (x5) and (x2) indicate the dilution factors. | | | | | |

The result of the reading using the following 5 different concentrations (0.1-1.0 µM) of each inhibitor allowed obtaining a straight line of concentrations against enzymatic activity. The concentration necessary to deduce the 50% enzymatic activity (IC₅₀) was calculated from the equation of said line.

| **Compound** | **IC₅₀** |
|---|---|
| MTC-141 | 0.18 µM |
| MTC-128 | 0.35 µM |
| MTC-126 | 0.85 µM |

## Claims

1. Compound of general formula 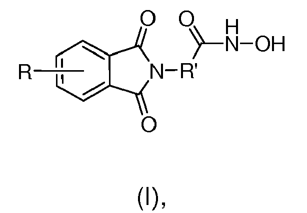 wherein
R' represents a -(CH₂)ₙ- radical wherein n is 5 or 6 or a 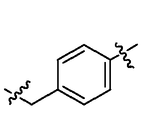 radical and R represents a hydrogen, C1-C3 alkyl, phenyl, benzyl, F, Cl, Br, -OR1 wherein R1 represents hydrogen, C1-C3 alkyl, phenyl or benzyl, -N02, an amine of formula -NR1R2 wherein R1 and R2, the same or different, can be hydrogen, C1-C3 alkyl, phenyl, or benzyl, or an -NHCOR3 radical wherein R3 can be C1-C3 alkyl, phenyl or benzyl optionally in the form of one of the salts thereof, particularly one of the pharmaceutically acceptable salts thereof, or one of the corresponding solvates thereof, with the proviso that when R is hydrogen, R' is different from -(CH₂)ₙ- wherein n is 5 or 6.

2. Compound according to claim 1, wherein R represents a C1-C3 alkyl, phenyl, benzyl, F, Cl, Br, -OR1 wherein R1 represents hydrogen, C1-C3 alkyl, phenyl or benzyl, -N02, an amine of formula -NR1R2 wherein R1 and R2, the same or different, can be hydrogen, C1-C3 alkyl, phenyl, or benzyl, or an -NHCOR3 radical wherein R3 can be C1-C3 alkyl, phenyl or benzyl, and R' represents a group -(CH₂)ₙ- wherein n is 5 or 6.

3. Compound according to claim 2, wherein R represents C1-C3 alkyl, F, Cl, Br, -OR1 wherein R1 is C1-C3 alkyl, phenyl or benzyl, -N02, NR1R2 wherein R1 is hydrogen and R2 represents a C1-C3 alkyl group, phenyl or benzyl, or -NHCOR3 wherein R3 is C1-C3 alkyl, phenyl or benzyl and R' represents a -(CH₂)ₙ- radical wherein n is 5 or 6.

4. Compound according to claim 3, wherein R represents a methyl, ethyl, F, Cl, Br, -OCH₃, -OPh, -OBn, -NO2, -NHR2 wherein R2 represents a C1-C3 alkyl group, phenyl or benzyl, or -NHCOCH₃, NHCOPh and R' represents a -(CH₂)ₙ- radical wherein n is 5 or 6.

5. Compound according to claim 4, wherein R represents a methyl, ethyl or N02 and R' represents a -(CH₂)ₙ- radical wherein n is 5 or 6.

6. Compound according to any of the previous claims, wherein R' represents a -(CH₂)ₙ- radical wherein n is 5 or 6 and the R substituent is located in position 5 of the phthalimide

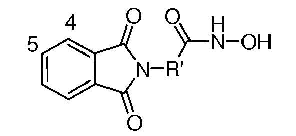

7. Compound according to claim 1, wherein R represents hydrogen, C1-C3 alkyl, phenyl, benzyl, F, Cl, Br, -OR1 wherein R1 represents hydrogen, C1-C3 alkyl, phenyl or benzyl, -N02, an amine of formula -NR1R2 wherein R1 and R2, the same or different, can be hydrogen, C1-C3 alkyl, phenyl, or benzyl, or an - NHCOR3 radical wherein R3 can be C1-C3 alkyl, phenyl or benzyl, and R' represents a 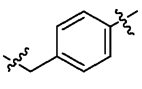 group.

8. Compound according to claim 7, wherein R represents hydrogen, C1-C3 alkyl, F, Cl, Br, -OR1 wherein R1 is C1-C3 alkyl, phenyl or benzyl, -N02, NR1R2 wherein R1 is hydrogen and R2 represents a C1-C3 alkyl group, phenyl or benzyl, or -NHCOR3 wherein R3 is C1-C3 alkyl, phenyl or benzyl and R' represents a 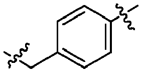 group.

9. Compound according to claim 8, wherein R represents, hydrogen, methyl, ethyl, F, Cl, Br, -OCH₃, -OPh, -OBn, -N02, -NHR2 wherein R2 represents a C1-C3 alkyl group, phenyl or benzyl, or -NHCOCH₃, NHCOPh and R' a 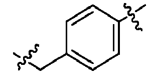 radical.

10. Compound according to any of claims 7 to 9, wherein R' represents a 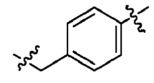 group and the R substituent, different from hydrogen, is located in position 5 of the phthalimide

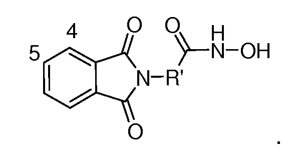

11. Compound according to claim 1 selected from the following group:
[1 ] 6-(5-methyl-1,3-dioxoisoindol-2-yl)-N-hydroxyhexanamide (MTC-141)
[2] 6-(5-nitro-1,3-dioxoisoindol-2-yl)-N-hydroxyhexanamide (MTC-127)
[3] 4-[(1,3-dioxo-1,3-dihydro-isoindol-2-yl)-methyl]-N-hydroxybenzamide (MTC-126).

12. A pharmaceutical composition containing one or more compounds of general formula (I) or one of the pharmaceutically acceptable salts thereof, or one of the corresponding solvates thereof according to any of the previous claims and one or more pharmaceutically acceptable excipients.

13. Compound of general formula (I) or one of the pharmaceutically acceptable salts thereof, or one of the corresponding solvates thereof according to any of claims 1 to 11, for the treatment and/or prophylaxis of diseases sensitive to the inhibition of histone deacetylases in a mammal, including a human being.

14. Compound of general formula (I) or one of the pharmaceutically acceptable salts thereof, or one of the corresponding solvates thereof according to any of claims 1 to 11, for the treatment and/or prophylaxis of cancer, particularly leukemia, solid and altered hormonal signal-dependent tumors, psoriasis, inflammatory type diseases, infection caused by HIV, Alzheimer's disease and senile dementia in a mammal, including a human being.

15. Use of a compound of general formula (I) or one of the pharmaceutically acceptable salts thereof, or one of the corresponding solvates thereof according to any of claims 1 to 11, in the production of a medicament for the treatment and/or prophylaxis of diseases sensitive to the inhibition of histone deacetylases in a mammal, including a human being.

16. Use of a compound of general formula (I) or one of the pharmaceutically acceptable salts thereof, or one of the corresponding solvates thereof according to any of claims 1 to 11, in the production of a medicament for the treatment and/or prophylaxis of cancer, particularly leukemia, solid and altered hormonal signal-dependent tumors, psoriasis, inflammatory type diseases, infection caused by HIV, Alzheimer's disease and senile dementia in a mammal, including a human being.

17. Process according to claim 1 for preparing a compound of general formula (I) 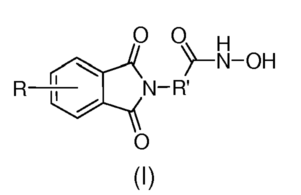 wherein
R' represents a -(CH₂)ₙ- radical wherein n is 5 or 6; and
R represents a C1-C3 alkyl, phenyl, benzyl, F, Cl, Br, -OR1 wherein R1 represents hydrogen, C1-C3 alkyl, phenyl or benzyl, -N02, an amine of formula -NR1R2 wherein R1 and R2, the same or different, can be hydrogen, C1-C3 alkyl, phenyl, or benzyl, or an -NHCOR3 radical wherein R3 can be C1-C3 alkyl, phenyl or benzyl,
comprising the following reaction steps:
step A: 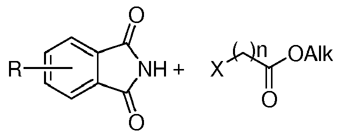 comprising reacting a derivative of phthalimide wherein R represents a C1-C3 alkyl, phenyl, benzyl, F, Cl, Br, -OR1 wherein R1 represents hydrogen, C1-C3 alkyl, phenyl or benzyl, -N02, an amine of formula -NR1R2 wherein R1 and R2, the same or different, can be hydrogen, C1-C3 alkyl, phenyl, or benzyl, or an -NHCOR3 radical wherein R3 can be C1-C3 alkyl, phenyl or benzyl,
with an ester derivative compound wherein
n is 5 or 6;
X represents a leaving group, selected from Br, Cl, -OSO₂CH₃ and -OSO₂Ph(pCH₃); and
Alk represents an alkyl group selected from ethyl, methyl and t-butyl, or an acid function protecting group;
in the presence of an inorganic base and in an inert solvent;
step B comprising the hydrolysis of the ester derivative obtained in step A of general formula 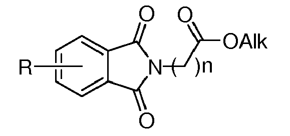 wherein n, Alk and R have the aforementioned meaning,
or alternatively, the elimination of the acid function protecting group to obtain the corresponding carboxylic acid derivative of general formula: 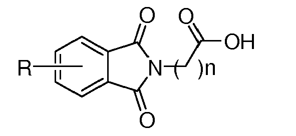 wherein R and n have the aforementioned meaning,
step C comprising contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin of general formula: 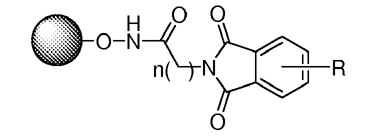 wherein n and R have the aforementioned meaning; and
step D comprising the release of the hydroxamic acid derivative bound to the Wang resin obtained in step C to obtain a compound of general formula (I) 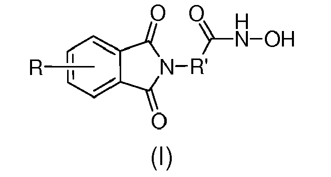 wherein R and R' have the previously defined meaning.

18. Process according to claim 1 for preparing a compound of general formula (I) 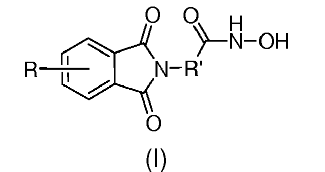 wherein R' is a 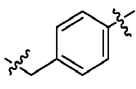 radical and
R represents hydrogen, C1-C3 alkyl, phenyl, benzyl, F, Cl, Br, -OR1 wherein R1 represents hydrogen, C1-C3 alkyl, phenyl or benzyl, -N02, an amine of formula -NR1R2 wherein R1 and R2, the same or different, can be hydrogen, C1-C3 alkyl, phenyl, or benzyl; or an -NHCOR3 radical wherein R3 can be C1-C3 alkyl, phenyl or benzyl,
comprising the following reaction steps:
step A : 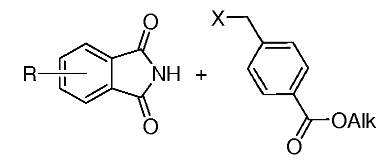 comprising reacting a derivative of phthalimide, wherein R represents hydrogen, C1-C3 alkyl, phenyl, benzyl, F, Cl, Br, -OR1 wherein R1 represents hydrogen, C1-C3 alkyl, phenyl or benzyl, -N02, an amine of formula -NR1R2 wherein R1 and R2, the same or different, can be hydrogen, C1-C3 alkyl, phenyl, or benzyl, or an -NHCOR3 radical wherein R3 can be C1-C3 alkyl, phenyl or benzyl,
with an ester derivative compound wherein
X represents a leaving group selected from Br, Cl, -OSO₂CH₃ and -OSO₂Ph(pCH₃); and
Alk represents an alkyl group selected from ethyl, methyl and t-butyl, or an acid function protecting group;
in the presence of an inorganic base and in an inert solvent;
step B comprising the hydrolysis of the ester derivative obtained in step A of general formula: 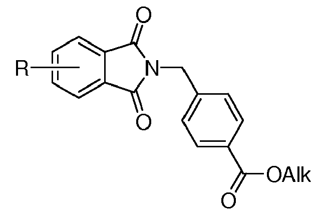 wherein R and Alk have the aforementioned meaning,
or alternatively, the elimination of the acid function protecting group to obtain the corresponding carboxylic acid derivative of general formula: 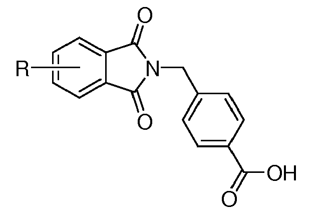 wherein R has the aforementioned meaning,
step C comprising contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin of general formula: 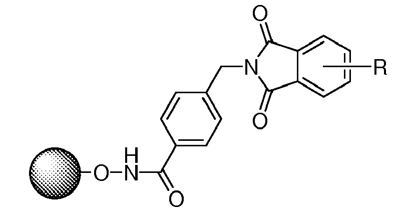 wherein R has the aforementioned meaning; and
step D comprising the release of the hydroxamic acid derivative bound to the Wang resin obtained in step C to obtain a compound of general formula (I) 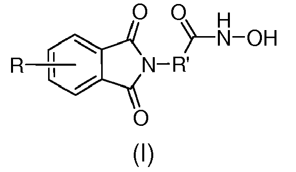 wherein R and R' have the previously defined meaning.
